# EUROPEAN PATENT APPLICATION

(11) **EP 3 406 241 A1**
(43) Date of publication of application: **28.11.2018**
(21) Application number: 17741356.4
(22) Date of filing: 16.01.2017
(51) Int. Cl.: A61K 9/20, A61K 47/10, A61K 47/32, A61K 47/38

(54) **INTRAORAL RETENTIVE DISINTEGRATING SOLID PREPARATION, METHOD FOR PRODUCING SAME AND POWDER COMPOSITION USED IN SAID PRODUCTION METHOD**

(30) Priority: 18.01.2016 JP 2016007031
(71) Applicant: Daicel Corporation, Osaka-shi, Osaka 530-0011 (JP)
(72) Inventor: HAMASAKI Momoko, Himeji-shi Hyogo 671-1281 (JP); HIRAMURA Takahiro, Tokyo 108-8230 (JP); OKABAYASHI Tomohito, Himeji-shi Hyogo 671-1281 (JP); SAKAGUCHI Anan, Himeji-shi Hyogo 671-1281 (JP)
(74) Representative: J A Kemp
(86) International application number: PCT/JP2017/001279
(87) International publication number: WO 2017/126478

(57) **Abstract**

The purpose of the present invention is to provide an intraoral retentive disintegrating solid preparation having excellent disintegrability and cohesiveness, a method for the production thereof, and a powder composition for use in the method for the said production.

The present invention relates to an intraoral retentive disintegrating solid preparation comprising at least one kind of a disintegrator and at least one kind of a water-soluble polymer, wherein a disintegration starting time is within 40 seconds and a cohesiveness index is 0.4 or more, a method for the production thereof, and a powder composition for use in the method for the said production, etc.

## Description

### Technical Field

The present invention relates to an intraoral retentive disintegrating solid preparation, which will rapidly become into pasty so as to be easily retained in an oral cavity, to a method for the production of the preparation and to powder composition to be used in the method, and the like.

### Background Art

As a method for administrating by absorption via intraoral mucosa of active ingredients such as an agent for treating allergy , which have been administered by subcutaneous injection and the like, there have been expected a sublingual administration-type liquid preparation; a solid preparation that will gradually disintegrate in the oral cavity; and an oral solid preparation such as a tablet and a troche for an oral care use such as a remedy of stomatitis, which will initiate its disintegration in the oral cavity after the lapse of an appropriate time.

For example, as a viscous liquid preparation is known a remedy of allergen-immunotherapy wherein pollen extract of Japanese cedar is sublingually administered. A patient will drop sublingually the pollen extract by him/herself once a day, and swallow it after having kept it under the tongue for about two minutes. Such preparation contains concentrated glycerin and sodium chloride and the like.

Also, as a sublingual tablet for desensitization therapy (allergen-immunotherapy) of allergic rhinitis due to Dermatophagoides antigen is known, for example, mite sublingual tablets named "Ashitea" and "Mitekyua" (in Japanese), which will disintegrate in from 10 seconds to a few minutes in the oral cavity.

Furthermore, a disintegrating sublingual tablet is known as a remedy of anginal attack. It will disintegrate in about 34 seconds after having been sublingually administered, so that nitroglycerin contained therein as an active ingredient will directly act on coronary vessels to show the effect of dilation of the coronary vessels usually in one or two minutes. It contains lactose hydrate, gum arabic, talc, corn starch and the like.

As other examples of the solid preparation that will gradually disintegrate in the oral cavity, there can be listed an intraoral mucosa adhesive preparation (a patch-type preparation) wherein a medicine will be absorbed through the intraoral mucosa into blood (PTL 1). This preparation is characterized by having a layer structure consisting of a mucosa-attaching layer, a supporting layer, two medicine layers and an intermediate layer.

Another intraoral adhesive preparation is known, which is characterized by having a plain or concave surface with gently-sloping "R" on one side, and a convex surface with gently-sloping "R" on the other side for gradually releasing the active ingredient in the oral cavity (PTR 2).

As a remedy of stomatitis and glossitis, there is also known a film-shaped patch that is attached in the oral cavity so as to protect a lesion from stimulus and continuously keep the action of an active ingredient. The patch contains poly acrylic acid, tri-ethyl citrate, hypromellose, ethyl cellulose, castor oil, titanium oxide and the like. There is also an ointment-type remedy of stomatitis and glossitis, which will be retained in the oral cavity for 30-60 minutes.

### Related Arts

### Patent Literatures

PTL 1: JP-A-2010-138124
PTL 2: JP-A-2011-210817

### Summary of Invention

### Problems to be solved by the Invention

In the prior arts, however, the viscous liquid preparation dropped sublingually and the sublingual tablet are actually difficult to be retained under the tongue. The intraoral adhesive preparation such as the film-shaped patch is difficult to attach, and will be easily peeled in such an attached site as the back of a lower lip. Furthermore, since the ointment-type preparation is applied in the oral cavity by hands, there are a problem from a sanitary point of view, and a problem that it is hard to apply in public.

Accordingly, an object of the present invention is to solve such technical problems as mentioned above, and to provide an intraoral retentive disintegrating solid preparation, which will rapidly disintegrate into the paste-state due to saliva in the oral cavity, so that it can be easily retained in the oral cavity.

### Means to Solve the Problem

Thus, the present invention relates to the following aspects.

### [Aspect 1]

An intraoral retentive disintegrating solid preparation comprising at least one kind of a disintegrator and at least one kind of a water-soluble polymer, wherein a disintegration starting time is within 40 seconds and a cohesiveness index is 0.4 or more.

### [Aspect 2]

The solid preparation according to Aspect 1, comprising carmellose as the disintegrator and sodium carmellose as the water-soluble polymer.

### [Aspect 3]

The solid preparation according to Aspect 1 or 2, comprising carmellose and crospovidone as the disintegrator, and sodium carmellose as the water-soluble polymer.

### [Aspect 4]

The solid preparation according to any one of Aspects 1-3, further comprising sugar alcohol or sugar, and a water-insoluble polymer.

### [Aspect 5]

The solid preparation according to Aspect 4, comprising D-mannitol as the sugar alcohol, and crystalline cellulose as the water-insoluble polymer.

### [Aspect 6]

The solid preparation according to any one of Aspects 1-5, further comprising a medicinal ingredient.

### [Aspect 7]

The solid preparation according to any one of Aspects 1-6, which is a tablet having hardness of from 20N or more.

### [Aspect 8]

A method for the production of the intraoral retentive disintegrating solid preparation according to any one of Aspects 1-7, which is characterized by compression-molding a powder composition in a dry process.

### [Aspect 9]

The method according to Aspect 8, wherein the water-soluble polymer and the medicinal ingredient are added and mixed with granulated substance comprising components other than the water-soluble polymer.

### [Aspect 10]

A powder composition for use in the method for the production according to Aspect 8 or 9, comprising a disintegrative particulate composition comprising at least one kind of the disintegrator and at least one kind of the water-soluble polymer.

### Advantages of Invention

The intraoral retentive disintegrating solid preparation according to the present invention is easy to handle as it is solid, so that it can be easily retained after the administration at a desired site in the oral cavity. Furthermore, as shown in Examples of the present specification, since it has a superior disintegrability, it will start disintegration within 40 seconds due to the absorption of saliva in the oral cavity, so as to rapidly soften into a paste-state. It will and further keep its shape and state as a mass without dispersing due to its excellent cohesiveness.

Since the intraoral retentive disintegrating solid preparation according to the present invention does not require such a special layer structure or surface configuration as seen in the prior arts, it can be easily produced with a simple process.

### Brief Description of Drawings

[Fig. 1] It shows the results of measurement of disintegrability of the intraoral retentive disintegrating solid preparation according to the present invention (Examples 1-5) and Comparative Examples 1 and 2
[Fig. 2] It shows the results of measurement of the cohesiveness of Example 1.
[Fig. 3] It shows the results of measurement of the cohesiveness of Example 2.
[Fig. 4] It shows the results of measurement of the cohesiveness of Example 3.
[Fig. 5] It shows the results of measurement of the cohesiveness of Example 4.
[Fig. 6] It shows the results of measurement of the cohesiveness of Example 5.
[Fig. 7] It shows the results of measurement of the cohesiveness of Comparative Example 1.
[Fig.8] It shows an outline of the measurement of disintegrability.
[Fig. 9] It shows an outline of the measurement of cohesiveness.

### Description of Embodiments of the Invention

The present invention relates to the intraoral retentive disintegrating solid preparation comprising at least one kind of a disintegrator and at least one kind of a water-soluble polymer, wherein a disintegration starting time is within 40 seconds, preferably within 35 seconds, more preferably within 30 seconds, much more preferably within 10 seconds; and a cohesiveness index is 0.4 or more, preferably 0.45 or more, more preferably 0.5 or more.

The "disintegration starting time" here means a time necessary for the swelling to reach a peak value (shown as "Peak Time" in Table 2) when it is measured under the conditions described in the present specification. The faster (shorter) the disintegration starting time is, the more excellent (higher) the disintegrability is. The "cohesiveness" is a property characterized by a value (index) obtained by a measuring method described in the present specification. As the value increases, the cohesiveness of the preparation becomes high so that the preparation will hardly disperse.

As demonstrated by the Examples, the intraoral retentive disintegrating solid preparation according to the present invention has the excellent disintegrability and cohesiveness. Accordingly, it will start disintegration within 40 seconds due to the absorption of saliva in the oral cavity, so that it can rapidly soften into the paste-state, and further keep its shape and state as the mass at the attached site without being dispersed. The "paste-state" generally means a flowable semi-solid state.

Any one or more disintegrators known to those skilled in the art can be used as the disintegrator comprised in the disintegrative particulate composition for use in the production of the solid preparation of the present invention. For example, it may comprise one or more components selected from acid-type carboxymethylcellulose (carmellose), crospovidone, croscarmellose sodium, low substituted hydroxypropylcellulose, carboxymethylcellulose calcium, various kinds of starch and processed starch such as starch sodium glycolate and hydroxypropyl starch. Additionally, crospovidone is a common name for a cross-linked polymer of 1-vinyl-2-pyrrolidone, and croscarmellose sodium is a common name for a cross-linked product of carboxymethylcellulose sodium.

Among the above disintegrators, carmellose, crospovidone, croscarmellose sodium, low substituted hydroxypropylcellulose, carboxymethylcellulose calcium and the like are water-insoluble polymers. The starch such as corn starch, potato starch, waxy corn starch, water-insoluble α-starch or water-insoluble partially α-starch; and the processed starch such as starch sodium glycolate and hydroxypropyl starch are also water-insoluble polymers.

Carmellose and/or crospovidone is preferred as the disintegrator.

The water-soluble polymer comprised in the above composition may be one or more compounds known for those skilled in the art such as those selected the group consisting of sodium carboxylmethylcellulose (also, called "sodium carmellose"), sodium alginate, carrageenan, xanthan gum and gelatin. The water-soluble polymer may be naturally-occurring or synthetic one. Sodium carmellose is a preferred water-soluble polymer.

It is preferred that the composition may further comprise as an excipient any sugars or sugar alcohols and inorganic compounds known in the art, such as calcium phosphate, water-containing silicon dioxide, calcium phosphate anhydrate, calcium hydrogen phosphate anhydrate, aluminum metasilicate, magnesium silicate, light anhydrous silicic acid, magnesium oxide and calcium silicate.

The sugars or sugar alcohols include D-mannitol, mannitol, erythritol, xylitol, trehalose, lactose, maltose, maltitol, glucose, sucrose, fructose, mannose, and sorbitol. As the sugars or sugar alcohols, two or more types of compounds properly selected from these compounds can also be used.

Moreover, as preferable examples thereof, there may be listed one or more compounds selected from the group consisting of D-mannitol, lactose, trehalose, cellulose, xylitol and calcium phosphate.

The composition may further comprise as an auxiliary excipient any water-insoluble polymer known in the art as long as it can effectively improve moldability of the composition. It may be naturally-occurring or synthetic one.

Preferable examples of the water-insoluble polymer include micro-fibrillated cellulose, crystalline cellulose, powdered cellulose and various kinds of cellulose derivatives.

Any crystalline cellulose and/or powdered cellulose known in the art can be used. As typical examples of the crystalline cellulose may be mentioned commercially-available products such as "Avicel" (FMC Corporation), "CEOLUS" (Asahi Kasei Chemicals Corp.), and "VIVAPUR" (RETTENMAIER) can be mentioned. As typical examples of the powdered cellulose, commercially-available products such as KC Flock (NIPPON PAPER Chemicals CO., LTD) and ARBOCEL (RETTENMAIER) and Solka Flock (Kimura Sangyo Co., Ltd.)

Furthermore, in addition to the above-described components, various types of optional components known to those skilled in the art may be properly added and mixed into the composition, for example, for the purpose of adjusting various characteristics such as the disintegrating force, binding force and ease in taking the tablet, without impairing the effects of the present invention by the above-described components. Examples of such components include other binding agents (auxiliary excipients) and functional additives such as a fluidizing agent.

As the fluidizing agent, for example, there may be mentioned crystalline cellulose, corn starch, light anhydrous silicic acid, water-containing silicon dioxide, magnesium metasilicate aluminate, magnesium stearate, calcium stearate, stearic acid, alumina magnesium hydroxide and talc.

There is no limitation on the form, shape and the like of the intraoral retentive disintegrating solid preparation according to the present invention, and the solid preparation may include any form and shape known in the art, such as a tablet, and film-type and a sheet-type solid preparation. The tablet is especially preferred. Furthermore, the intraoral retentive disintegrating solid preparation may optionally comprise a suitable mount of any medicinal (active) ingredient.

An amount of each component comprised in the solid preparation of the present invention can be optionally determined by those skilled in the art, depending on, for example, the type of the components, and the type and use of the preparation. In general, relative to the total weight of the preparation, the amount of the disintegrator is in a range of 1% to 70% by weight, the amount of the water-soluble polymer is in a range of 0.1% to 30% by weight. The contents of the excipients, the water-insoluble polymer, binding agents (auxiliary excipients) and fluidizing agents may be optionally determined depending on their purposes.

Since the intraoral retentive disintegrating solid preparation according to the present invention has the excellent disintegrability and cohesiveness in the oral cavity, it can be used as the solid preparation that will gradually disintegrate in the oral cavity; and pharmaceuticals such as a preparation for the intraoral mucosa absorption, which are used for various uses and purposes such as the tablet and the troche for an oral care use such as a remedy of stomatitis. Additionally, it is used as various foods such as supplemental foods, nutrition function foods and health foods, and oral care products such as tooth paste.

The solid preparation according to the present invention may optionally comprise any other components depending on its use and purpose.

It may comprise for the use of pharmaceuticals, for example, in addition to a medicinal ingredient, other components acceptable as additives from a pharmaceutical point of view, such as surfactants, lubricants, acidulants, sweeteners, corrigents, flavoring agents, colorants, and stabilizing agents, when needed. As these optional components, for example, appropriate ingredients described in "Japanese Pharmaceutical Excipients Directory" (YAKUJI NIPPO LIMITED) or the Japanese Pharmacopoeia. There is no limitation in the kind of the medicinal ingredient and the above auxiliary agents. Also, the blending ratios of the disintegrative particulate composition, the medicinal ingredient and each optional ingredient (component) are not particularly limited as long as the expected effects of the present invention are brought about, and the blending ratios can properly be determined by those skilled in the art. An application or kind of the medicinal ingredients comprised in the present invention may include, for example, agents affecting each organ such as the central nervous system, peripheral nervous system, a sensory organ, a circulatory organ, a respiratory organ and a digestive organ and an urogenital organ; hormone drug; agents affecting metabolism such as a vitamin drug, an analeptic, an agent affecting blood and body fluid; agents affecting the function of tissue and cell such as an agent activating cellular function, an agent affecting tumors, an radioactive medicine, an anti-allergic agent; medicines based on a medical prescription relating to herbal medicines and Chinese medicines; antibiotics; agents for chemotherapy, biological drug; agents for pathogenic organisms such as parasites; agents for formulation use, diagnosis, public health and in-vitro diagnosis.

It may comprise for the use of the foods and oral care, for example, various nutritional components such as proteins, carbohydrates, lipids and minerals; various vitamins and their derivatives; and designated or existing additives according to Food Sanitation Law, Art. 10; and other components acceptable as a food component (a food additive) listed in a list of general additives for food and drink, such as acidulants, sweeteners, excipients, surfactants, lubricants, corrigents, flavoring agents, colorants, and stabilizing agents. It may also comprise other optional components that are accepted for quasi - drugs, which are listed in ingredient specification of the quasi - drugs of The Law on Securing Quality, Efficacy and Safety of Products Including Pharmaceuticals and Medical devices (previous "Pharmaceutical Affairs Law", Art.2.

The intraoral retentive disintegrating solid preparation according to the present invention can be produced by any methods known to those skilled in the art. For example, it may be produced by compression-molding the powder composition comprising the disintegrative particulate composition in a dry process. For example, it may be easily prepared by adding the water-soluble polymer and the medicinal ingredient to the granulated substance comprising components other than the water-soluble polymer and mixing them.

Accordingly, the present invention also relates to a method for the production of the intraoral retentive disintegrating solid preparation, and to a powder composition for use in the above method for the production, which comprises a disintegrative particulate composition comprising at least one kind of the disintegrator and at least one kind of the water-soluble polymer.

The powder composition can be also easily produced by any method known to those skilled in the art. The granule comprising the disintegrators, excipients, binders and the like may be prepared in advance as a premix-type additive composition before it is mixed with other materials.

The additive composition can be produced by mixing each of the components all together by any method known to those skilled in the art .

Alternatively, the powder composition may be produced by various granulation processes. Any granulation method may be used, and a dry granulation process and a wet granulation process may be used to produce the powder composition.

The dry granulation process comprises the steps of mixing each powder components comprised in the powder composition optionally with an appropriate binder and the like, breaking the resulting mixture into small bulks with a high pressure, and appropriately crushing and granulating them. Examples of the dry granulation process include crushing granulation and roll-compressing method.

The wet granulation process is a method in which each component is dispersed in the presence of water, and the dispersion is dried to form complexes. As specific examples of the wet granulation process can be mentioned spray methods such as spray drying, tumbling granulation, agitation granulation and fluidized-bed granulation; the freeze-drying method; kneading granulation, and the like. The composition can be produced by any of these methods known to a person skilled in the art.

The intraoral retentive disintegrating solid preparation according to the present invention in a form of the tablet has a hardness of 20N or more, preferably 30N or more, more preferably 40N or more. As long as its disintegrability is not seriously deteriorated, there is actually no upper limit for the hardness, which is 140N, 130N or 120N, for example.

In addition, contents of all related art documents cited in the present specification are incorporated herein by reference.

Hereinafter, the present invention will more specifically be described with reference to Examples. However, the present invention is not considered to be limited to the Examples.

### [Evaluation on average particle size, water content, hardness, disintegrability and cohesiveness]

The average particle size and water content of the powder composition; and hardness, disintegrability and cohesiveness of the tablets obtained in the Examples and Comparative Examples were measured based on the following conditions/methods.

### Average particle size:

5 g of the powder composition was subjected to a measurement with a Φ75 mm automatic shaking sieve device (Type "M-3T", Tsutsui Scientific Instruments Co., Ltd.).

### Water content:

5 g of the powder composition was subjected to a measurement using a halogen water content measuring device (Type "HB43", METTLER TOLEDO K.K.).

### Hardness:

a hardness (N) was measured with a digital Kiya hardness tester (Fujiwara Scientific Company Co., Ltd.). The measurements were repeated six times, respectively, and average values thereof were regarded as measurement results.

### Disintegrability:

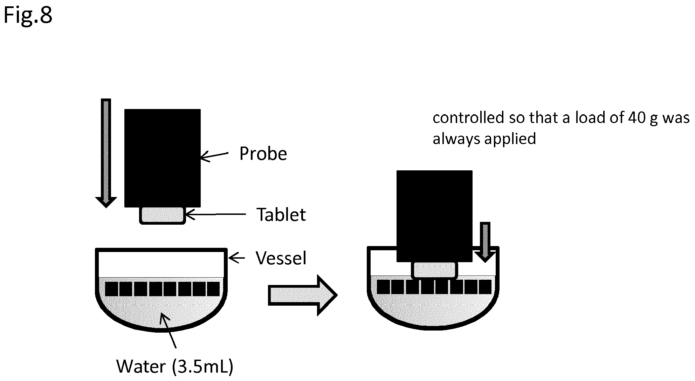

It was measured with a special jig, Tablet Disintegration Rig (A/TDR) of a texture analyzer (TA, XT plus, Stable Microsystems, Inc.). Its outline is shown in Fig. 8.

A tablet was attached with a double sided tape to the lowest end of a probe connected to a load cell. The measurement was started at position 0 mm to which the probe had moved down so that the tablet came into contact with a vessel of A/TDR containing 3.5 mL of water. While the position of the probe was controlled so that a load of 40 g could be always applied during the measurement, and change of the position of the probe was then measured. The time required for the swelling (the position of the probe) to reach a maximum is taken as the "disintegration starting time" (peak time) . The measurements were repeated six times, respectively, and average values thereof were regarded as measurement results.

### Cohesiveness:

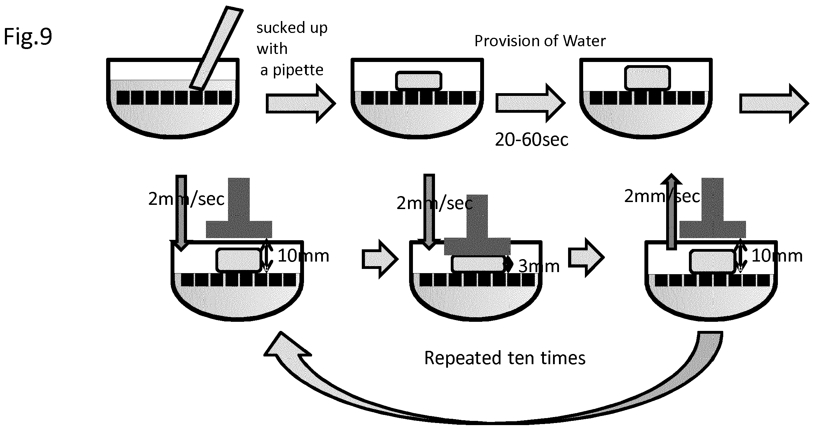

It was measured with the vessel of the special jig, Tablet Disintegration Rig (A/TDR) of the texture analyzer (TA, XT plus, Stable Microsystems, Inc.), and a cylinder-type resin probe with a diameter of 20 mm. Its outline is shown in Fig. 9.

Water in the vessel of A/TDR was sucked up with a pipette from the upper surface of the special jig having holes, so that water contained in the bottom of the vessel would be provided only through the holes. After water had been absorbed into the whole of the tablet set in the vessel (about from 20 seconds to one minute later), stress was measured every time when the tablet was compressed by the cylinder-type resin probe ten times continuously, which had moved down for 7 mm at 2mm/sec from 10 mm high of the lowest end of the tablet. A value obtained by dividing the stress measured at tenth time by that measured at sixth time was considered as a cohesiveness index. The measurements were repeated six times, respectively, and average values thereof were regarded as measurement results.

### Examples

### [Examples 1-5]

### [Production of disintegrative particulate composition]

280 g of mannitol (D-mannitol, Merck Ltd.), 75 g of carmellose (NS-300, GOTOKU CHEMICAL CO., LTD.), 100 g of crystalline cellulose (CEOLUS PH-101, Asahi Kasei Chemicals Corp.) and 40 g of crospovidone (Polyplasdone INF-10, ISP Japan) were charged to a fluidized-bed granulator (LAB-1, Powrex Corporation), and 300 g of purified water was sprayed onto the resulting mixture at a rate of 12 g/minute to thereby obtain granulated substance. The resulting granulated substance had the following values for physical properties: (1) an average particle size of 93 microns and (2) a water content of 2.3% by weight.

### [Production of intraoral retentive disintegrating solid preparation]

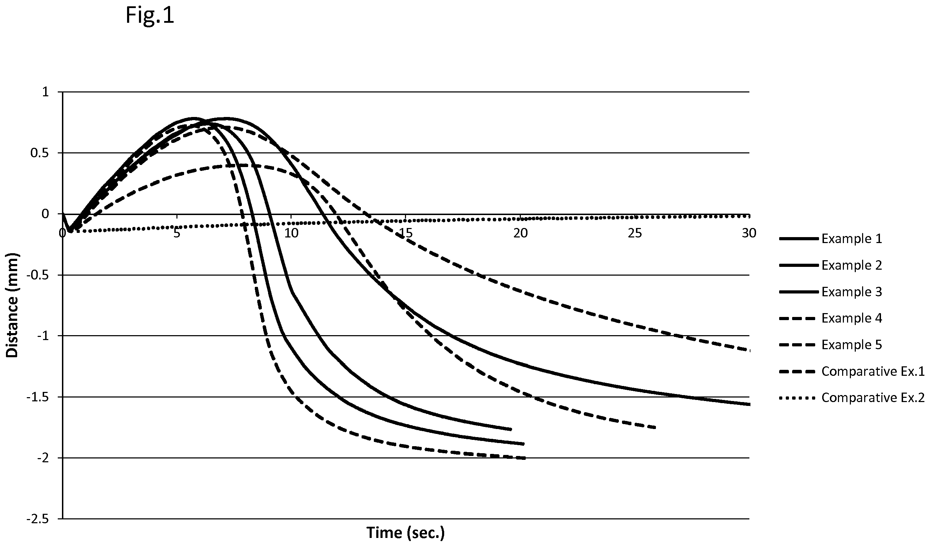

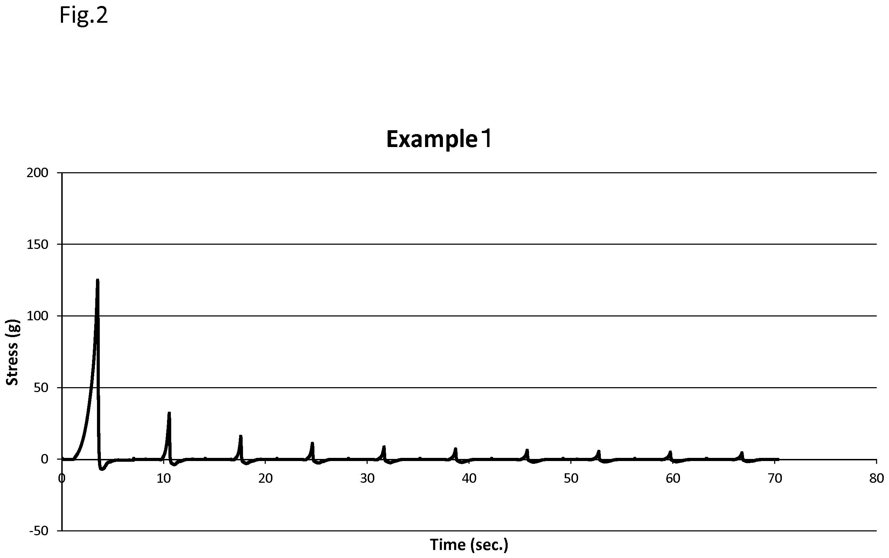

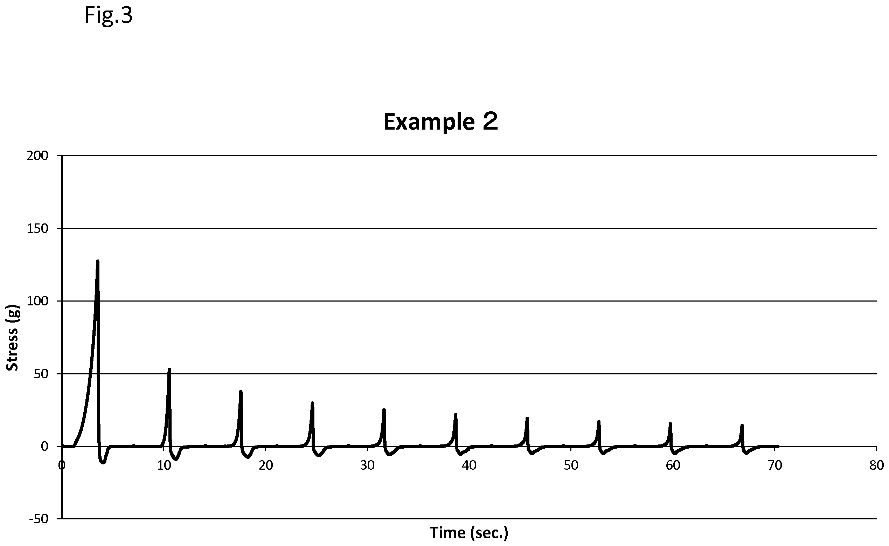

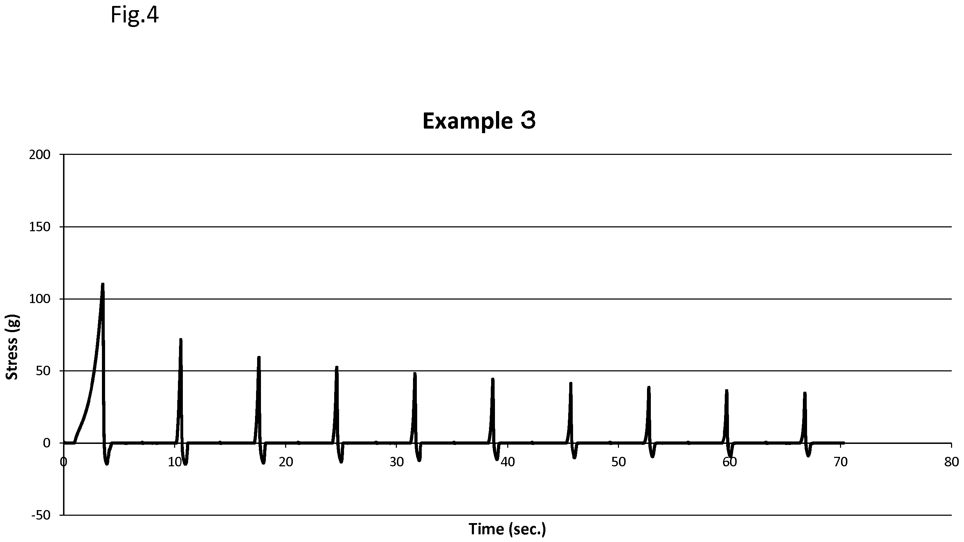

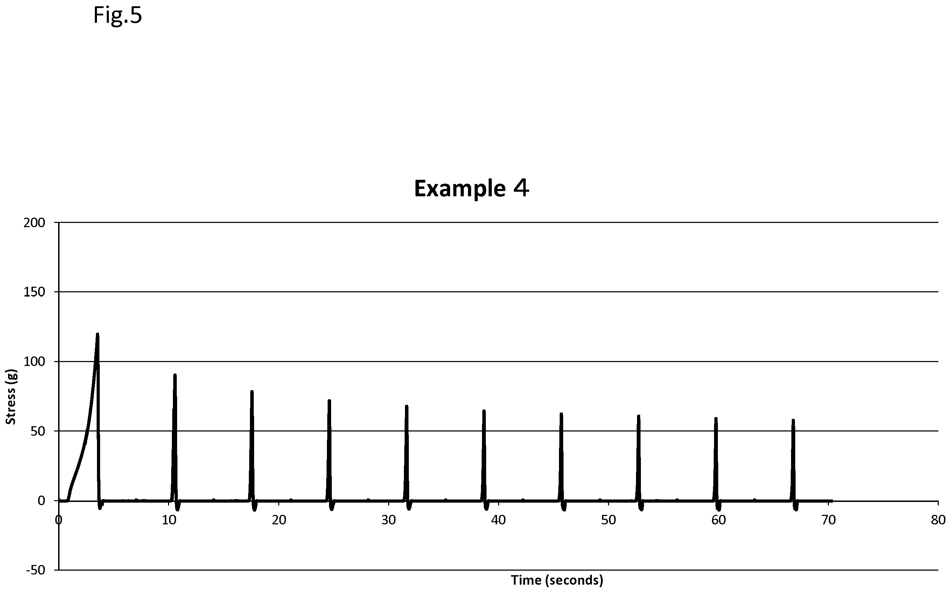

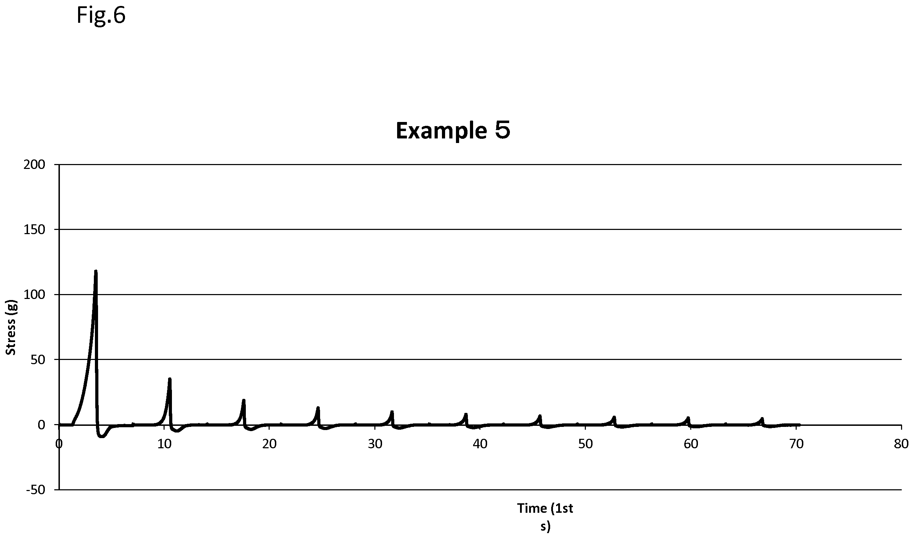

98.5-99.7 parts by weight of the resulting granulated substance and 1.5-0.3 parts by weight of sodium carmellose (CMC Daicel, Daicel FineChem Ltd.) were mixed. 88.5 parts by weight of the resulting granulated substance, 1.5 parts by weight of sodium carmellose (CMC Daicel, Daicel FineChem Ltd.) and 10 parts by weight of vitamin C (Vitamin C, Iwaki Seiyaku Co.,Ltd) were mixed. Each of the resulting mixture was then subjected to tableting with asimple tableting machine (HANDTAB-100, ICHIHASHI-SEIKI Co., Ltd.) to thereby obtain the intraoral retentive disintegrating solid preparation according to the present invention as a flat faced tablet having a diameter of 8.0 mm and a weight of 250 mg, wherein a small amount of magnesium stearate (Taihei Chemical Industrial Co. Ltd.) had been applied as a lubricant in advance to a mortar inner surface, and the surfaces of an upper-pestle and a lower-pestle in the above tableting machine, and a tablet compression force was controlled to yield tablet hardness of about 100N. Their composition and hardness are shown in Table 1. The measurement results of Disintegrability and Cohesiveness are shown in Figure 1 and Table 2, and Figures 2-6 and Table 3, respectively.

### [Comparative Example 1]

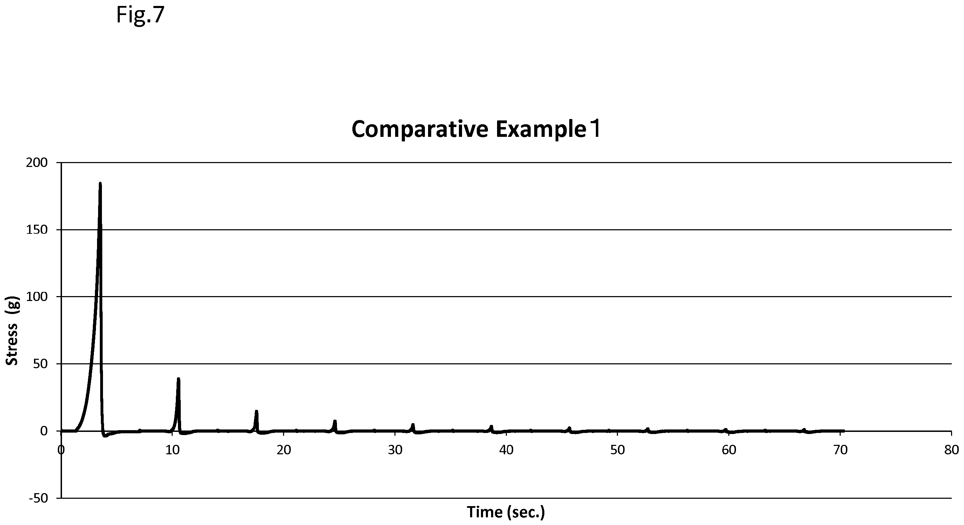

The granulated substance obtained in each Example was then subjected to tableting with the simple tableting machine (HANDTAB-100, ICHIHASHI-SEIKI Co., Ltd.) to thereby obtain a flat faced tablet (Comparative Example 1) having a diameter of 8.0 mm and a weight of 250 mg, wherein a small amount of magnesium stearate (Taihei Chemical Industrial Co. Ltd.) had been applied as the lubricant in advance to the mortar inner surface, and the surfaces of the upper-pestle and the lower-pestle in the above tableting machine, and a tablet compression force was controlled to yield tablet hardness of about 100N. Their composition and hardness are shown in Table 1. The measurement results of Disintegrability and Cohesiveness are shown in Figure 1 and Table 2, and Figure 7 and Table 3, respectively.

### [Comparative Example 2]

69.0 parts by weight of lactose (FlowLac90, MEGGLE JAPAN CO.,LTD.), 29.5 parts by weight of corn starch (granule corn starch, JAPAN CORN STARCH CO., LTD.) and 1.5 parts by weight of sodium carmellose (CMC Daicel, Daicel FineChem Ltd.) were mixed. The resulting mixture was then subjected to tableting with the simple tableting machine (HANDTAB-100, ICHIHASHI-SEIKI Co., Ltd.) to thereby obtain a flat faced tablet (Comparative Example 2) having a diameter of 8.0 mm and a weight of 250 mg, wherein a small amount of magnesium stearate (Taihei Chemical Industrial Co. Ltd.) had been applied as the lubricant in advance to the mortar inner surface, and the surfaces of the upper-pestle and the lower-pestle in the above tableting machine, and a tablet compression force was controlled to yield tablet hardness of about 100N. Their composition and hardness are shown in Table 1. The measurement result of Disintegrability is shown in Figure 1. Cohesiveness could not be measured since the tablet did not disintegrate.

**[Table 1]**

| | Ex.1 | Ex.2 | Ex.3 | Ex.4 | Ex.5 | Comparative Ex.1 | Comparative Ex.2 |
|---|---|---|---|---|---|---|---|
| Granulated Substance | 99.7 | 99.5 | 99.0 | 98.5 | 88.5 | 100 | - |
| CMC-Na | 0.3 | 0.5 | 1.0 | 1.5 | 1.5 | - | 1.5 |
| Vitamin C | - | - | - | - | 10 | - | - |
| Lactose | - | - | - | - | - | - | 69.0 |
| Corn Starch | - | - | - | - | - | - | 29.5 |
| Tablet Compression Force (kN) | 7 | 8 | 8 | 7 | 10 | 7 | 9 |
| Hradness (N) | 98 | 100 | 98 | 90 | 101 | 102 | 107 |

**[Table 2]**

| | Peak Position mm | Peak Time sec |
|---|---|---|
| Ex. 1 | 0.79 | 5.8 |
| Ex. 2 | 0.74 | 6.3 |
| Ex. 3 | 0.79 | 7.1 |
| Ex. 4 | 0.71 | 7.0 |
| Ex. 5 | 0.40 | 7.9 |
| Comparative Ex. 1 | 0.73 | 5.6 |
| Comparative Ex. 2 | - | - |

The results shown in Figure 1 and Table 2 demonstrate that the tablet prepared in Examples 1-5 and Comparative Example 1 started disintegration within 10 seconds after the absorption of water followed by swellin under the conditions described therein. On the other hand, it was shown that the tablet obtained in Comparative Example 2 did not swell or disintegrate even after the passage of 10 minutes from the contact with water.

**[Table 3]**

| | Stress Compression (g) at | | | | | | |
|---|---|---|---|---|---|---|---|
| | 6th | 7th | 8th | 9th | 10th | Average | Cohesiveness (Index) |
| Ex.1 | 7.2 | 6.3 | 5.5 | 5.0 | 4.6 | 5.7 | 0.64 |
| Ex. 2 | 21.8 | 19.3 | 17.1 | 15.5 | 14.5 | 17.7 | 0.66 |
| Ex. 3 | 44.3 | 41.4 | 38.5 | 36.3 | 34.6 | 39.0 | 0.78 |
| Ex. 4 | 64.8 | 62.6 | 60.9 | 59.4 | 57.9 | 61.1 | 0.89 |
| Ex. 5 | 8.1 | 6.8 | 5.9 | 5.3 | 4.8 | 6.2 | 0.59 |
| Comparative Ex. 1 | 3.4 | 2.3 | 1.7 | 1.3 | 1.0 | 1.9 | 0.29 |

The results shown in Figures 2-7 and Table 3 demonstrate that although the tablets obtained in Examples 1-5 would soften due to the absorption of water, they showed high cohesiveness measured with repeated compression, meaning that they could easily maintain their shape or form. On the other hand, the tablet obtained in Comparative Example 1 showed low cohesiveness measured with repeated compression after having softened due to the absorption of water, meaning that they would immediately disperse.

The tablets obtained in Examples 1-5 and Comparative Examples 1 and 2 were taken in mouth by three adults of both sexes. Although the tablet obtained in Examples 1-5 softened due to saliva in the oral cavity, they retained their state as the mass without being dispersed. On the other hand, the tablet obtained in Comparative Examples 1 rapidly disintegrated without retaining its original form, and the tablet obtained in Comparative Examples 2 maintained its hardness even after the contact with saliva in the oral cavity.

### [Comparative Example 3]

10 parts by weight of vitamin C (Vitamin C, Iwaki Seiyaku Co.,Ltd) was mixed with 90 parts by weight of the granulated substance obtained in Example. The resulting mixture was then subjected to tableting with the simple tableting machine (HANDTAB-100, ICHIHASHI-SEIKI Co., Ltd.) to thereby obtain a flat faced tablet (Comparative Example 3) having a diameter of 8.0 mm and a weight of 250 mg, wherein a small amount of magnesium stearate (Taihei Chemical Industrial Co. Ltd.) had been applied as the lubricant in advance to the mortar inner surface, and the surfaces of the upper-pestle and the lower-pestle in the above tableting machine, and a tablet compression force was controlled to yield tablet hardness of about 100N.

### [Comparative Example 4]

50 parts by weight of glycerin (Nichi-Iko Pharmaceutical Co., Ltd.), 10 parts by weight of sodium chloride (Wako Pure Chemical Corporation) and 40 parts by weight of purified water were mixed to prepare solution. 5 g of vitamin C (Vitamin C, Iwaki Seiyaku Co., Ltd.) was then solved into 45 g of the solution to give dropping liquid as Comparative Example 4.

The tablets obtained in Example 5 and Comparative Example 3 and the 1 mL of the dropping liquid (Comparative Example 4) were taken under the tongue by six adults of both sexes, and a retainable time under the tongue was measured. While the tablet of Comparative Example 3 showed the retainable time of 27.3 seconds in average, the tablet obtained in Example 5 showed the retainable time of 61.7 seconds in average, demonstrating that the retention time under the tongue was prolonged. As the dropping liquid obtained in Comparative Example 4 spread over the whole of the mouth immediately after it had been taken, it was difficult to be retained under the tongue.

### Industrial Applicability

The present invention significantly contributes to research and development of the solid preparation that will gradually disintegrate in the oral cavity; and pharmaceuticals such as a solid preparation for the intraoral mucosa absorption , which are used for various uses and purposes such as the tablet and the troche for an oral care use such as a remedy of stomatitis.

## Claims

1. An intraoral retentive disintegrating solid preparation comprising at least one kind of a disintegrator and at least one kind of a water-soluble polymer, wherein a disintegration starting time is within 40 seconds and a cohesiveness index is 0.4 or more.

2. The solid preparation according to Claim 1, comprising carmellose as the disintegrator and sodium carmellose as the water-soluble polymer.

3. The solid preparation according to Claim 1 or 2, comprising carmellose and crospovidone as the disintegrator, and sodium carmellose as the water-soluble polymer.

4. The solid preparation according to any one of Claims 1-3, further comprising sugar alcohol or sugar, and a water-insoluble polymer.

5. The solid preparation according to Claim 4, comprising D-mannitol as the sugar alcohol, and crystalline cellulose as the water-insoluble polymer.

6. The solid preparation according to any one of Claims 1-5, further comprising a medicinal ingredient.

7. The solid preparation according to any one of Claims 1-6, which is a tablet having hardness of from 20N or more.

8. A method for the production of the intraoral retentive disintegrating solid preparation according to any one of Claims 1-7, which is **characterized by** compression-molding a powder composition in a dry process.

9. The method according to Claim 8, wherein the water-soluble polymer and the medicinal ingredient are added and mixed with granulated substance comprising components other than the water-soluble polymer.

10. A powder composition for use in the method for the production according to Claim 8 or 9, comprising a disintegrative particulate composition comprising at least one kind of the disintegrator and at least one kind of the water-soluble polymer.
